# EUROPÄISCHE PATENTSCHRIFT

(11) **EP 3 817 793 B1**
(45) Veröffentlichungstag und Bekanntmachung des Hinweises auf die Patenterteilung: **07.02.2024**
(21) Anmeldenummer: 19737494.5
(22) Anmeldetag: 02.07.2019
(51) Int. Cl.: A61M 1/36

(54) **VERFAHREN ZUM AUTOMATISIERTEN PRIMEN EINES EXTRAKORPORALEN BLUTLEITUNGSSYSTEMS SOWIE EINE VORRICHTUNG HIERFÜR**
METHOD FOR AUTOMATING THE PRIMING OF AN EXTRACORPOREAL BLOOD-CIRCUIT AND DEVICE TO CARRY OUT THE METHOD
PROCÉDÉ POUR AUTOMATISER L'AMORÇAGE D'UN CIRCUIT EXTRACORPOREL DU SANG ET DISPOSITIF POUR UN TEL PROCÉDÉ

(30) Priorität: 03.07.2018 DE 102018116071
(43) Veröffentlichungstag der Anmeldung: 12.05.2021
(73) Patentinhaber: B. Braun Avitum AG, 34212 Melsungen (DE)
(72) Erfinder: RITTER, Kai-Uwe, 34212 Melsungen (DE); BRÖGGER, Sebastian, 34593 Knüllwald (DE)
(74) Vertreter: Winter, Brandl - Partnerschaft mbB
(86) Internationale Anmeldenummer: PCT/EP2019/067684
(87) Internationale Veröffentlichungsnummer: WO 2020/007829

(56) Entgegenhaltungen:
- US-A- 5 776 091
- US-A1- 2012 103 902

## Beschreibung

Die Erfindung betrifft ein Verfahren zum Primen eines extrakorporalen Blutsystems für eine Behandlung mittels einer Vorrichtung zur extrakorporalen Blutbehandlung, wobei das extrakorporale Blutsystem eine arterielle Blutleitung, eine venöse Blutleitung, ein Pumpsegment zum Zusammenwirken mit einer peristaltischen Blutpumpe der Vorrichtung und eine Blutbehandlungseinheit, insbesondere einen Dialysator, umfasst.

Sie betrifft außerdem eine Vorrichtung zur extrakorporalen Blutbehandlung mit einer peristaltischen Blutpumpe zum Fördern von Flüssigkeit durch peristaltisches Verformen eines Pumpsegments, eines extrakorporalen Blutsystems mit einer arteriellen Blutleitung, einer venösen Blutleitung, dem Pumpsegment und einer Blutbehandlungseinheit, insbesondere einem Dialysator, wobei die Blutpumpe eine teilkreisförmige Führungsfläche und einen um eine in Wesentlichen horizontale Drehachse drehangetriebenen Rotor aufweist, der derart mit der Führungsfläche zusammenwirkt, dass in dem zwischen der Führungsfläche und dem Rotor angeordneten Pumpsegment eine Querschnittsverengung ausbildet wird, welche Vorrichtung zur Durchführung des erfindungsgemäßen Verfahrens besonders geeignet ist.

### Hintergrund der Erfindung

Vor einer extrakorporalen Blutbehandlung, wie zum Beispiel einer Dialyse, muss Luft aus dem sogenannten extrakorporalen Blutsystem, im Wesentlichen bestehend aus Blutschläuchen (Blutschlauchsatz) und einem Dialysator, entfernt werden. Dies geschieht, indem im System befindliche Luft durch Einleiten einer Flüssigkeit (Primingflüssigkeit) verdrängt und möglichst vollständig entfernt wird. Dieser Vorgang wird im Allgemeinen als Primen oder Priming bezeichnet.

Als Primingflüssigkeit wird entweder sterile Kochsalzlösung aus einem Beutel oder bei sogenannten Online-Maschinen mit zwei Dialysierflüssigkeitsfiltern ultrareine Dialysierflüssigkeit aus der Maschine verwendet. Die Anforderungen an die Reinheit der Primingflüssigkeit sind sehr hoch, da verständlicherweise Verschmutzung und Unsterilität des Systems vermieden werden müssen und das Primingvolumen üblicherweise dem Patienten beim Anlegen als Bolus in den Blutkreislauf infundiert wird. Aus dem gleichen Grund sollte keine oder nur sehr wenig Luft in der Primingflüssigkeit vorhanden sein.

Zum Primen eines extrakorporalen Blutsystems gibt es im Stand der Technik unterschiedliche Verfahren. Bei einem bekannten Verfahren, das auch als manuelles Priming- oder Single-Pass-Verfahren bezeichnet wird, werden die arteriellen und venösen Blutleitungen des extrakorporalen Blutsystems zunächst in der für die Behandlung bestimmten Weise an die Blutbehandlungsvorrichtung (Dialysemaschine) angeschlossen. Das bedeutet auch, dass zumindest eine der Leitungen in eine Blutpumpe der Vorrichtung eingelegt und okkludiert wird, das heißt in einer zur Förderung von Flüssigkeit durch die Pumpe erforderlichen Weise zusammengequetscht wird. In einer Reihe von manuell durchzuführenden Schritten werden die arterielle Blutleitung, die venöse Blutleitung und der Dialysator dann mit Primingflüssigkeit gefüllt, wobei die Primingflüssigkeit mittels einer Pumpaktion der Pumpe in und durch das extrakorporale Blutsystem geleitet wird.

In einem ähnlichen Verfahren, das auch als manuelles Gravity-Priming bezeichnet wird, wird die Blutleitung durch die Pumpe zunächst nicht okkludiert und die Primingflüssigkeit durch Wirkung der Schwerkraft in das System eingebracht, bis das Pumpsegment der Blutleitung mit Primingflüssigkeit gefüllt ist. Zum Beispiel kann das Pumpsegment des nicht mit Flüssigkeit gefüllten Schlauchs nicht-okklusiv in die Pumpe eingehängt werden. Dabei dient die Pumpe lediglich als Halter, das Pumpsegment könnte auch an anderer Stelle hängen, beispielsweise an einem Infusionsständer oder ähnlichem. Ein Ende einer mittels einer Klemme abgeklemmten arteriellen Schlauchleitung wird zum Befüllen mit einem Kochsalzbeutel verbunden. Das gegenüberliegende Ende der Schlauchleitung wird hoch gehalten, um ein Herauslaufen von Primingflüssigkeit, hier Kochsalzlösung, zu verhindern. Dann wird die Klemme geöffnet und Kochsalzlösung strömt aus dem Beutel in das zu primende Schlauchsegment. Erst nach dem luftfreien Befüllen des Pumpsegments wird dieses in die Blutpumpe eingefädelt und okkludiert. Mit einer Reihe von manuell durchzuführenden Schritten wird danach die Primingflüssigkeit mittels einer Pumpaktion der Blutpumpe in und durch das extrakorporale Blutsystem geleitet. Dazu wird das gefüllte arterielle Schlauchsegment mit der Blutseite des Dialysators verbunden. Danach wird das venöse Schlauchsegment mit dem Dialysator verbunden und der Dialysator vom arteriellen Anschluss her bei laufender Blutpumpe gefüllt. Wenn die gesamte Blutleitung und der Dialysator komplett mit Primingflüssigkeit gefüllt sind, werden die patientenseitigen Anschlüsse der Blutleitung miteinander verbunden. Die Blutpumpe wird gestartet und die Primingflüssigkeit im extrakorporalen System zirkuliert. Im nächsten Schritt wird der Dialysator über seine Dialysierflüssigkeitsanschlüsse mit Dialysierflüssigkeit gefüllt. Nachfolgend können die Pegel der Primingflüssigkeit in der arteriellen Schlauchleitung und in der venösen Schlauchleitung gesetzt werden, indem aus einer Tropfkammer Luft zum Beispiel über Serviceleitungen abgelassen wird. Das extrakorporale Blutsystem ist nun vollständig mit Primingflüssigkeit gefüllt, die darin zirkuliert wird, bis der Patient ankommt und die eigentliche Behandlung beginnt.

### Stand der Technik

Aus der WO 2008/077 573 A2 ist ein Verfahren zum Primen eines Blutschlauchsatzes mit einer venösen und einer arteriellen Leitung bekannt, deren patientenseitige Anschlüsse mit zwei separaten Eingängen einer Kammer, insbesondere eines Beutels, in Verbindung stehen, und deren maschinenseitige Anschlüsse mit einem Dialysator in Verbindung stehen. Das Verfahren umfasst die Schritte eines parallelen Befüllens sowohl der venösen als auch der arteriellen Leitung über eine Zuleitung, so dass Primingflüssigkeit durch beide Eingänge in die Kammer fließt, sowie eines Zirkulierens der Primingflüssigkeit in dem Kreislauf aus den Leitungen, dem Dialysator und der Kammer über eine Pumpe, so dass einer der Eingänge der Kammer als Eingang und der andere als Ausgang fungiert.

Aus der WO 1996/040 320 A1 ist ein Verfahren zum Vorfüllen einer Dialysemaschine bekannt, die einen Dialysator, eine Pumpe und einen Blutschlauchsatz, der eine arterielle Leitung zum Ansaugen von Blut von einem Patienten, einen Pumpenkopf für die Pumpe zum Pumpen des Blutes zum Dialysator, eine venöse Leitung zum Zurückleiten des durch den Dialysator gepumpten Blutes zum Patienten und einen Verbinder zum Verbinden der arteriellen Leitung und der venösen Leitung enthält, besitzt, wobei das Verfahren die folgenden Schritte umfasst:
Verbinden der arteriellen Leitung und der venösen Leitung mit dem Verbinder;
Verbinden des Verbinders mit einer Ablaufleitung;
Schalten eines Ablaufventils zwischen den Verbinder und die Ablaufleitung;
Befüllen der arteriellen Leitung mit einer sterilen Lösung;
Öffnen des Ablaufventils und einer arteriellen Klemme an der arteriellen Leitung, damit die sterile Lösung in der arteriellen Leitung durch den Verbinder abläuft und am offenen Ablaufventil vorbei die Ablaufleitung nach unten fließt;
Schließen der arteriellen Klemme;
Öffnen einer venösen Klemme an der venösen Leitung und Betreiben der Pumpe in einer Vorwärtsrichtung, um sterile Lösung von der arteriellen Leitung durch den Pumpenkopf und den Dialysator anzusaugen und um der sterilen Lösung zu ermöglichen, durch die venöse Leitung und den Verbinder an dem offenen Ablaufventil vorbei die Ablaufleitung nach unten abzulaufen; und
Schließen des Ablaufventils, Öffnen der arteriellen Klemme und Betreiben der Pumpe, um die sterile Lösung durch den Dialysator und den Blutschlauchsatz zurückzuleiten.

Die US 5 776 091 A offenbart ein Verfahren zum automatischen Primen und Rezirkulieren von steriler Flüssigkeit durch einen extrakorporalen Kreislauf eines Dialysegeräts mit einer Blutpumpe, einem Dialysator und einem Blutschlauchsatz, der eine arterielle Leitung zum Abführen von Blut von einem Patienten und eine venöse Leitung zum Zurückführen des Bluts zu dem Patienten umfasst. Das Dialysegerät öffnet und schließt dabei selektiv Klemmen an den arteriellen und venösen Leitungen und betätigt ferner ein Abfallventil zum selektiven Öffnen und Schließen einer Abfallleitung, die zu einem Abfallablauf führt. Ein Anschluss verbindet sowohl die arterielle als auch die venöse Leitung mit der Abfallleitung stromabwärts der arteriellen und venösen Klammern und stromaufwärts des Abfallventils. Das Dialysegerät betätigt automatisch die Blutpumpe, die Klemmen und das Abfallventil, um den Diaylsator und das Blutschlauchset mit der sterilen Flüssigkeit zu spülen und die verbrauchte Flüssigkeit in den Abfallablauf zu leiten. Das Dialysegerät betätigt dann die Blutpumpe und die Klemmen, um den Dialysator und das Blutschlauchset erneut mit zusätzlicher steriler Flüssigkeit zu füllen und die sterile Flüssigkeit ohne Unterstützung eines Bedieners des Dialysegeräts durch den extrakorporalen Kreislauf zurückzuführen.

Ein wesentlicher Nachteil bekannter Priming-Verfahren ist, dass in der Regel eine gewisse Menge Luft im extrakorporalen Leitungssystem eingeschlossen bleibt, die im Verlauf des Primings beispielsweise durch Klopfen, Schütteln oder ähnlich manuelle, vom Bedienpersonal gewissenhaft durchzuführende Arbeiten entfernt werden muss. Zwar stellt das Gravity Priming eine besonders gute Möglichkeit dar, Luft nahezu vollständig aus dem extrakorporalen Blutsystem zu entfernen. Allerdings ist es derzeit nicht zu automatisieren, da beim automatischen Priming das Pumpsegment der Blutschlauchleitung beim Aufrüsten bereits in die Blutpumpe eingelegt wird und bei bekannten Maschinen mit automatischer Einfädelfunktion das Pumpsegment in die Pumpe eingelegt und automatisch eingefädelt wird, sobald der Deckel der Pumpe geschlossen wird.

Ein Nachteil bei Verfahren, die kein Gravity-Priming anwenden, ist, dass nach Füllen des Systems die arterielle und die venöse Leitung verbunden werden und dann die Primingflüssigkeit zirkuliert wird, um restliche Luftblasen zu entfernen. Diese sammeln sich im Luftabscheider und senken dort den Pegel, wodurch ein zusätzlicher Arbeitsschritt erforderlich wird, weil der Pegel im Luftabscheider neu gesetzt werden muss.

Ein weiterer Nachteil bei bekannten automatisierten Verfahren kann Restluft darstellen, die im Pumpsegment verbleibt und durch die Pumpaktion der Pumpe zu Mikroblasen zerhackt bzw. verschäumt wird. Solche Mikroblasen sind unerwünscht, da sie unter Umständen zu Mikroembolien beim Patienten führen können. Außerdem können zusätzliche Mikroblasen unerwünschte Alarme auslösen. Schließlich ist es bei bekannten automatischen Verfahren infolge der Okklusion des Blutschlauchs nicht möglich, Drucksensoren vor und nach der Pumpe im Rahmen der Vorbereitung der Maschine zur Behandlung miteinander zu vergleichen und abzustimmen.

### Kurzbeschreibung der Erfindung

Ausgehend von dem vorstehend beschriebenen Stand der Technik liegt der Erfindung die Aufgabe zugrunde, die zuvor angeführten Nachteile zu beseitigen, insbesondere den Vorbereitungsprozess einer extrakorporalen Blutbehandlung, insbesondere einer Dialyse, zu verbessern und zumindest teilweise zu automatisieren, sowie eine Vorrichtung bereit zu stellen, die besonders zur Durchführung des erfindungsgemäßen Verfahrens geeignet ist.

Nach der Erfindung wird diese Aufgabe gelöst durch ein Verfahren zum Primen eines extrakorporalen Blutsystems für eine Behandlung mittels einer Vorrichtung zur extrakorporalen Blutbehandlung, wobei das extrakorporale Blutsystem eine arterielle Blutleitung, eine venöse Blutleitung, ein Pumpsegment zum Zusammenwirken mit einer peristaltischen Blutpumpe der Vorrichtung und eine Blutbehandlungseinheit, insbesondere einen Dialysator, umfasst, wobei ein Anschluss der venösen Blutleitung an einen venösen Blutanschluss der Blutbehandlungseinheit angeschlossen wird, ein Anschluss der arteriellen Blutleitung an einen arteriellen Blutanschluss der Blutbehandlungseinheit angeschlossen wird und das extrakorporale Blutsystem an ein Reservoir mit Primingflüssigkeit angeschlossen wird, nachfolgend das nicht okkludierte extrakorporale Blutsystem mit Primingflüssigkeit gefüllt wird und das Pumpsegment nach dem Füllen mit Primingflüssigkeit automatisch mittels einer Blutpumpe der Blutbehandlungsvorrichtung okkludiert wird. Man kann auch sagen, dass das extrakorporale Blutsystem in einem nicht okkludierten Zustand mit Primingflüssigkeit gefüllt wird und das Pumpsegment nach dem Füllen mit Primingflüssigkeit automatisch mittels der Blutpumpe okkludiert wird.

Diese Aufgabe wird außerdem gelöst durch eine Vorrichtung zur extrakorporalen Blutbehandlung mit einer peristaltischen Blutpumpe zum Fördern von Flüssigkeit durch peristaltisches Verformen eines Pumpsegments, eines extrakorporalen Blutsystems mit einer arteriellen Blutleitung, einer venösen Blutleitung, dem Pumpsegment und einer Blutbehandlungseinheit, insbesondere einem Dialysator, wobei die Blutpumpe eine teilkreisförmige Führungsfläche und einen um eine im Wesentlichen horizontale Drehachse drehangetriebenen Rotor aufweist, der derart mit der Führungsfläche zusammenwirkt, dass in dem zwischen der Führungsfläche und dem Rotor angeordneten Pumpsegment eine Querschnittsverengung ausbildet wird, wobei sich die Führungsfläche von einem Bereich unterhalb des Rotors teilweise um diesen herum bis zu einem Bereich oberhalb des Rotors erstreckt und zur Seite hin offen ist. Der Ausdruck "zur Seite hin offen" ist mit Bezug auf das Schwerefeld der Erde zu verstehen, wobei "unten" zur Erde hingerichtet, "oben" von der Erde abgewandt und "zur Seite hin" die Bereiche zwischen "oben" und "unten" bezeichnet. Infolge dieser Ausrichtung der Führungsfläche der Blutpumpe relativ zum Schwerefeld der Erde kann das Pumpsegment in vorteilhafter Weise derart in der Pumpe angeordnet werden, dass es zwingend von "unten" nach "oben" mit Primingflüssigkeit befüllt wird, so dass besonders einfach und effektiv luftfrei befüllt und geprimed werden kann. Zur Seite hin offen bedeutet in diesem Zusammenhang, dass die Führungsfläche die Rotorachse über einem Umhüllungswinkelbereich umgibt, zum Beispiel zwischen etwa 260° und 280°, vorzugsweise 270°, und der verbleibende Bereich von zwischen etwa 80° bis 100°, vorzugsweise 90°, offen ist, wobei dieser verbleibende Bereich mit Bezug auf die Horizontale und das Schwerefeld der Erde seitlich neben der Rotorachse angeordnet ist.

Bei der Blutpumpe kann es sich insbesondere um eine peristaltische Schlauchpumpe handeln. Sie kann ein Pumpengehäuse mit einer teilkreisförmigen Führungsfläche aufweisen, innerhalb der ein Rotor drehbar angetrieben angeordnet ist, um eine zwischen der Führungsfläche und dem Rotor befindliche Schlauchleitung zum Fördern von Flüssigkeit dadurch zusammen zu quetschen. Die Vorrichtung und/oder die Blutpumpe kann bzw. können außerdem eine Haltekontur aufweisen, zum nicht verschließenden / nicht okkludierenden Halten der Blutleitung(en). Die Blutpumpe ist vorzugsweise in oder an einer im Wesentlichen vertikalen Maschinenfront der Vorrichtung zur extrakorporalen Blutbehandlung angeordnet.

Die arterielle Blutleitung, die venöse Blutleitung und das Pumpsegment können im Rahmen der Erfindung insbesondere als elastisch verformbare Fluidleitungen oder Schläuche ausgebildet sein.

Man kann auch sagen, dass nach der Erfindung das extrakorporale Blutsystem zunächst vollständig aufgerüstet und an der Vorrichtung zur extrakorporalen Blutbehandlung angeordnet wird. Es kann sogar in der für eine Behandlung bestimmten Weise an der Vorrichtung angeschlossen werden, mit der Ausnahme, dass das Pumpsegment nicht okkludiert wird. Nicht okkludiert im Sinne der Erfindung heißt, dass das Pumpsegment von der Primingflüssigkeit durchströmbar ist, insbesondere ungehindert durchströmbar ist. Dies kann zum Beispiel dadurch erreicht werden, indem das Pumpsegment von der Blutpumpe nicht zusammengequetscht wird.

Vorteilhafte Ausführungsformen der Erfindung sind in den Unteransprüchen beansprucht und werden nachfolgend näher erläutert.

Eine Ausführungsform der Vorrichtung ist dadurch gekennzeichnet, dass das extrakorporale Blutsystem an ein Reservoir mit Primingflüssigkeit angeschlossen wird, indem ein patientenseitiger Anschluss der venösen Blutleitung oder ein patientenseitiger Anschluss der arteriellen Blutleitung oder eine damit fluidisch verbundene Füllleitung an das Reservoir mit Primingflüssigkeit angeschlossen wird. So kann das Blutsystem an sich vollständig aufgerüstet werden, also insbesondere die arterielle Blutleitung, die venöse Blutleitung und die Behandlungsvorrichtung in bestimmungsgemäßer Weise miteinander verbunden werden, so dass für das Primen und eine nachfolgende Behandlung in vorteilhafter Weise nur sehr wenige Handlungen durchzuführen sind.

Nach einer weiteren Ausführungsform wird der patientenseitige Anschluss der venösen Blutleitung an das Reservoir mit Primingflüssigkeit angeschlossen und nachfolgend das extrakorporale Blutsystem von arteriell nach venös gefüllt wird, wobei das Reservoir insbesondere ein Substituatport der Vorrichtung oder ein externer Behälter mit Primingflüssigkeit ist. Füllen von arteriell nach venös bedeutet in diesem Zusammenhang, dass die Primingflüssigkeit in die arterielle Blutleitung eingeleitet wird und von dort zur venösen Blutleitung durch das Blutsystem strömt. Zum besseren Entfernen von Luft aus dem System und der Primingflüssigkeit kann im weiteren Verlauf des Primings die Strömungsrichtung der Primingflüssigkeit im Blutsystem einmal oder mehrmals umgekehrt werden.

Eine Ausführungsform der Erfindung ist dadurch gekennzeichnet, dass der patientenseitige Anschluss der arteriellen Blutleitung an das Reservoir mit Primingflüssigkeit angeschlossen wird und nachfolgend das extrakorporale Blutsystem von venös nach arteriell befüllt wird, wobei das Reservoir insbesondere ein Substituatport der Vorrichtung oder ein externer Behälter mit Primingflüssigkeit ist. Füllen von venös nach arteriell bedeutet in diesem Zusammenhang, dass die Primingflüssigkeit in die venöse Blutleitung eingeleitet wird und von dort zur arteriellen Blutleitung durch das Blutsystem strömt. Dies ist entgegen der üblicherweise meist beim Primen verwendeten Richtung. Durch Primen von venös nach arteriell kann Luft komplett aus einer üblicherweise bei der Vorrichtung zur Blutbehandlung vorhandenen venösen Luftfalle verdrängt werden, da diese dann von unten befüllt wird. Dies ist besonders vorteilhaft für sogenannte luftfreie Blutleitungen. Zum besseren Entfernen von Luft aus dem System und der Primingflüssigkeit kann im weiteren Verlauf des Primings die Strömungsrichtung der Primingflüssigkeit im Blutsystem einmal oder mehrmals umgekehrt werden.

Nach einer anderen Ausführungsform der Erfindung kann vor dem Füllen mit Primingflüssigkeit ein patientenseitiger Anschluss der venösen Blutleitung oder ein patientenseitiger Anschluss der arteriellen Blutleitung (das heißt ein nicht an das Reservoir mit Primingflüssigkeit angeschlossener Anschluss) mit einem Brauchflüssigkeitsanschluss (Wasteport) der Vorrichtung zur extrakorporalen Blutbehandlung oder mit einem separaten Brauchflüssigkeitsreservoir, insbesondere einem Abfallbehälter oder Abfallbeutel, verbunden werden. Verbunden werden in diesem Sinne bedeutet strömungstechnisch und vorzugsweise nicht kontaktierend verbunden. Auf diese Weise kann überschüssige Primingflüssigkeit besonders einfach und hygienisch entsorgt werden.

Bei einer weiteren Ausführungsform der Erfindung kann das Pumpsegment vor dem Füllen mit Primingflüssigkeit derart in die Blutpumpe eingelegt werden oder relativ zu dieser positioniert wird, dass es während des Einfüllens von Primingflüssigkeit in das Blutsystem durch die Blutpumpe nicht okkludiert ist (und vorzugsweise während des Einfüllens von Primingflüssigkeit auch nicht okkludiert wird) und (erst) nach dem vollständigen Füllen des extrakorporalen Blutsystems mit Primingflüssigkeit okkludiert wird. Insbesondere kann das Pumpsegment automatisch durch Einschalten der Blutpumpe in deren Förderstrecke eingebracht und okkludiert werden. Dazu kann die Vorrichtung nach der Erfindung und/oder deren Blutpumpe zumindest eine Haltevorrichtung aufweisen, um das Pumpsegment in einer ein automatisches Einfädeln des Pumpsegments in die Blutpumpe ermöglichenden Weise nicht-okkludierend zu halten.

Alternativ kann das Pumpsegment vor dem Füllen mit Primingflüssigkeit in eine Förderstrecke der Blutpumpe eingelegt werden, wobei die Blutpumpe derart ausgebildet ist, dass das Pumpsegment während des Füllens des extrakorporalen Blutsystems mit Primingflüssigkeit durch die Blutpumpe nicht okkludiert ist und wird, und das Pumpsegment nach dem vollständigen Füllen des extrakorporalen Blutsystems mit Primingflüssigkeit in der Förderstrecke okkludiert wird. Dies kann zum Beispiel dadurch erzielt werden, indem die Blutpumpe als peristaltische Blutpumpe mit einem zur Führungsfläche relativpositionierbaren Rotor und/oder relativpositionierbaren Anpressrollen versehen ist, der/die sich beim Primen bzw. Befüllen des Blutsystems mit Primingflüssigkeit in einer das Pumpsegment nicht okkludierenden Stellung befinden und zum Fördern von Primingflüssigkeit in eine das Pumpsegment okkludierende Stellung relativ zur Führungsfläche positioniert werden können. Hinsichtlich der Vorrichtung nach der Erfindung ist anzumerken, dass der Rotor der Blutpumpe insbesondere relativ zur Führungsfläche in einer Richtung quer zu seiner Drehachse positionierbar und/oder kippbar sein kann und/oder zur Rotordrehachse in radialer Richtung relativpositionierbare Anpresselemente aufweisen kann. Nach einer Ausführungsform ist der Rotor der Pumpe zusammen mit einem Führungselement zum Führen des Pumpsegments in einer nicht okkludierenden Position relativ zur Führungsfläche positionierbar. Das Führungselement kann insbesondere oberhalb des Rotors und damit auch oberhalb des Pumpsegments angeordnet sein.

Nach der Erfindung kann die Blutpumpe während des Primens in einer Förderrichtung betrieben werden, die der Förderrichtung während einer Blutbehandlung entgegengesetzt ist. Dadurch kann in besonders einfacher Weise ein Befüllen des Blutsystems mit und/oder Durchströmen des Blutsystems von Primingflüssigkeit von venös nach arteriell erzielt werden, ohne dass dafür eine Umrüstung des Blutsystems an der Maschine erforderlich ist.

Nach einer Ausführungsform des erfindungsgemäßen Verfahrens wird das Blutsystem vor dem Füllen mit Primingflüssigkeit in einer zur Behandlung bestimmten Weise an die Vorrichtung zur extrakorporalen Blutbehandlung angeschlossen, mit der Ausnahme, dass das Pumpsegment nicht okkludiert wird. Das bedeutet, dass die Vorrichtung derart mit dem Blutsystem aufgerüstet wird, dass zum Durchführen einer Behandlung lediglich noch das Pumpsegment zu okkludieren ist. Auf diese Weise wird eine besonders effektive Möglichkeit zur Vorbereitung von extrakorporalen Blutbehandlungen geschaffen.

Im Rahmen der Erfindung kann insbesondere vor dem Füllen des extrakorporalen Blutsystems mit Primingflüssigkeit ein Sensortestlauf durchgeführt werden. Dies kann insbesondere erfolgen, indem die arterielle Blutleitung und die venöse Blutleitung verschlossen werden, in besonders einfacher Weise mittels Schlauchklemmen, und der Druck, insbesondere Luftdruck, im extrakorporalen Blutsystem erhöht wird. Außerdem können im Rahmen der Erfindung Drucksensoren nach dem Priming und sogar während der Behandlung wiederholt geprüft werden, indem bei nicht okkludiertem Pumpsegment Messwerte der Drucksensoren verglichen und überprüft werden. Dazu muss lediglich das Pumpsegment aus dem okkludierten Zustand in den nicht okkludierten Zustand überführt werden. Dies kann automatisch oder manuell durch eine Bedienperson erfolgen. Bei einer für ein automatisches Überführen in den nicht okkludierten Zustand geeigneten Fortbildung der Vorrichtung ist die Pumpe derart ausgebildet, dass das Pumpsegment jederzeit automatisiert aus der Förderstrecke entfernt und nach erfolgter Druckmessung wieder automatisiert in der Förderstrecke platziert werden kann. Alternativ kann dies durch eine vorstehend beschriebene Relativpositionierung von Anpresselementen des Rotors und/oder des Rotors relativ zur Führungsfläche erfolgen. Auf diese Weise ist es besonders einfach möglich, im Rahmen einer Behandlung eine Sensorüberprüfung durchzuführen, indem das Pumpsegment ausgefädelt / de-okkludiert wird, der Druck verglichen und evtl. korrigiert/kalibriert wird und dann das Pumpsegment wieder eingefädelt / okkludiert und die Behandlung fortgeführt wird.

Grundsätzlich kann nach der Erfindung die Primingflüssigkeit mittels Überdruck aus einem Substituatport der Vorrichtung oder mittels hydrostatischem Druck in das extrakorporale Blutsystem gefüllt werden.

Zusammenfassend kann man sagen, dass durch die Erfindung insbesondere die folgenden Vorteile und Verbesserungen erzielt werden können:
- besonders einfache Handhabung mit wenigen von Bedienpersonal durchzuführenden Schritten
- hochflexibel mit Möglichkeit eines Befüllens von venös nach arteriell und umgekehrt
- Verbesserte Luftentfernung beim Priming
- sehr weitgehend automatisierbar
- kann einen Abgleich von venösen und arteriellen Drucksensoren während des Priming ermöglichen und unterstützen
- Gegenüber einer Befüllung des Blutsystems nach Stand der Technik (von arteriell nach venös) kann ein Arbeitsschritt des Pegelsetzens in der Tropfkammer durch Entlüften der Kammer entfallen

### Kurzbeschreibung der Figuren

Die Erfindung wird im Folgenden anhand beispielhafter und in den angehängten Figuren gezeigter Ausführungsformen näher erläutert. Dabei zeigt:
Fig. 1 einen schematischen Schaltplan einer Vorrichtung zur extrakorporalen Blutbehandlung während einer Blutbehandlung,
Fig. 2 einen schematischen Schaltplan der Vorrichtung zur extrakorporalen Blutbehandlung der Figur 1 während einer ersten Sequenz einer Vorbereitung der Blutbehandlung (Priming),
Fig. 3 einen schematischen Schaltplan der Vorrichtung zur extrakorporalen Blutbehandlung der Figur 1 während einer zweiten Sequenz einer Vorbereitung der Blutbehandlung (Priming),
Fig. 4 eine Detaildarstellung einer Blutpumpe der Vorrichtung während der Vorbereitung und
Fig. 5 eine Detaildarstellung der Blutpumpe der Vorrichtung während der Blutbehandlung.

### Figurenbeschreibung

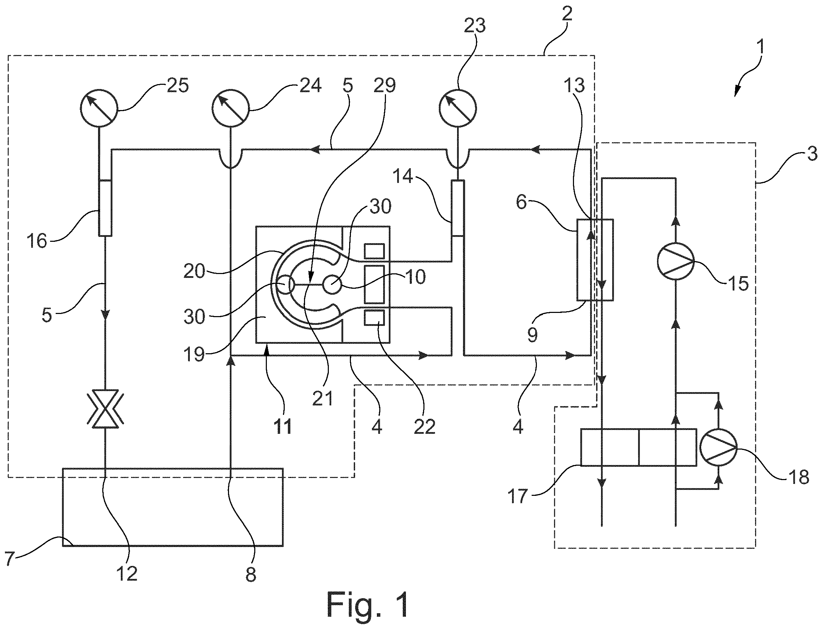

Fig. 1 zeigt in einem schematischen Schaltplan das Flüssigkeitssystem 1 einer Vorrichtung zur extrakorporalen Blutbehandlung, hier in Form einer Dialysevorrichtung, während einer Behandlung, wie zum Beispiel einer Hämodialyse, einer Hämofiltration oder eine Hämodiafiltration. Das Flüssigkeitssystem umfasst ein extrakorporales Blutsystem 2, auch als extrakorporaler Blutkreislauf bezeichnet, und einen Dialysierflüssigkeitskreislauf 3.

Das extrakorporale Blutsystem 2 besteht im Wesentlichen aus einer arteriellen Blutleitung 4, hier als arterielle Schlauchleitung 4 ausgebildet, einer venösen Blutleitung 5, hier als venöse Schlauchleitung 5 ausgebildet, und einer Blutbehandlungseinheit 6 in Form eines Filters 6 oder Dialysators 6. Während einer Blutbehandlung verbindet das extrakorporale Blutsystem 2 einen in Figur 1 angedeuteten Patienten 7 mit der Dialysevorrichtung.

Die arterielle Blutleitung 4 weist einen patientenseitigen Anschluss 8 in Form einer arteriellen Kanüle 8 sowie einen filterseitigen Anschluss 9 in Form eines Luer-Konnektors 9 auf. Außerdem umfasst die arterielle Blutleitung 4 ein Pumpsegment 10 zum Zusammenwirken mit einer Blutpumpe 11 der Blutbehandlungsvorrichtung. Die venöse Blutleitung 5 umfasst einen patientenseitigen Anschluss 12 in Form einer venösen Kanüle 12 sowie einen filterseitigen Anschluss 13 in Form eines Luer-Konnektors 13. Anhand von Strömungsrichtungspfeilen ist in Figur 1 angedeutet, dass dem Patienten 7 bei der Behandlung mittels der arteriellen Kanüle 8 Blut entnommen und mittels der Blutpumpe 11 durch die arterielle Blutleitung 4 über eine arterielle Luftfalle 14 zum Dialysator 6 gefördert wird. In diesem findet die eigentliche Behandlung, hier Reinigung, des Bluts statt. Vom Dialysator 6 strömt das Blut über die venöse Blutleitung 5 und eine venöse Luftfalle 16 zur venösen Kanüle 12 zurück in den Patienten 7. Der Dialysator 6 ist auch in den Dialysierflüssigkeitskreislauf 3 integriert und wird im Gegenstrom von Dialysierflüssigkeit durchströmt, die mittels einer Dialysierflüssigkeitspumpe 15 gefördert wird. Der Dialysierflüssigkeitskreislauf 3 weist außerdem eine Bilanzierungseinrichtung 17 und eine Ultrafiltrationspumpe 18 auf.

Die Blutpumpe 11 ist als peristaltische Schlauchpumpe 11 ausgebildet. Sie besitzt ein Pumpengehäuse 19 mit einer teilkreisförmigen Führungsfläche 20, einen innerhalb der teilkreisförmigen Führungsfläche 20 drehbar um eine im Wesentlichen horizontale Rotorachse oder Drehachse 29 angetriebenen Rotor 21 mit Anpresselementen 30 beispielsweise in Form von Anpressrollen 30 sowie eine Haltekontur 22 zum nicht verschließenden / nicht okkludierenden Halten der arteriellen Blutleitung 4. Wie aus den Figuren ersichtlich ist, ist das Pumpsegment 10 ein Teil der arteriellen Blutleitung 4, nämlich der Teil, der sich in der Blutpumpe 11 zwischen der Führungsfläche 20 und dem Rotor 21 befindet. Im Rahmen der Erfindung kann das Pumpsegment 10 außerdem als separate elastisch verformbare Fluidleitung ausgebildet sein, die beiderseits mit der arteriellen Blutleitung 4 verbunden ist. Das Pumpsegment 10 wird während der Blutbehandlung durch die Wirkung des Rotors 21 zwischen diesem und der Führungsfläche 20 des Pumpengehäuses 19 verformt und zusammengequetscht, das heißt okkludiert, derart, dass eine Fluidförderung von der Niederdruckseite der arteriellen Blutleitung 4 zu deren Hochdruckseite bewirkt wird (angedeutet durch Strömungsrichtungspfeile in Figur 1).

Die in den Figuren 1, 2 und 3 dargestellte Vorrichtung zur extrakorporalen Blutbehandlung kann außerdem nicht dargestellte weitere Messeinrichtungen, Pumpen, Blasenfänger usw. umfassen. Zur Überwachung von Therapievorgängen besitzt sie insbesondere Druckaufnehmer oder Drucksensoren 23, 24, 25. Im Einzelnen sind dies ein den Druck auf der Hochdruckseite der Blutpumpe 11 aufnehmender Eingangsdruckaufnehmer 23, ein arterieller Druckaufnehmer 24 zwischen der arteriellen Kanüle 8 und der Blutpumpe 11 sowie ein venöser Druckaufnehmer 25 im Bereich der venösen Luftfalle 16.

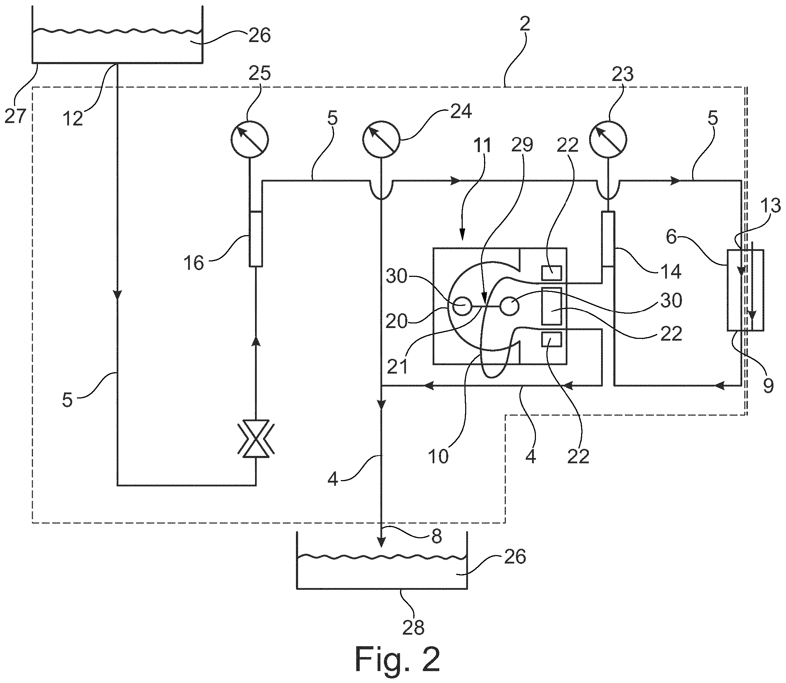

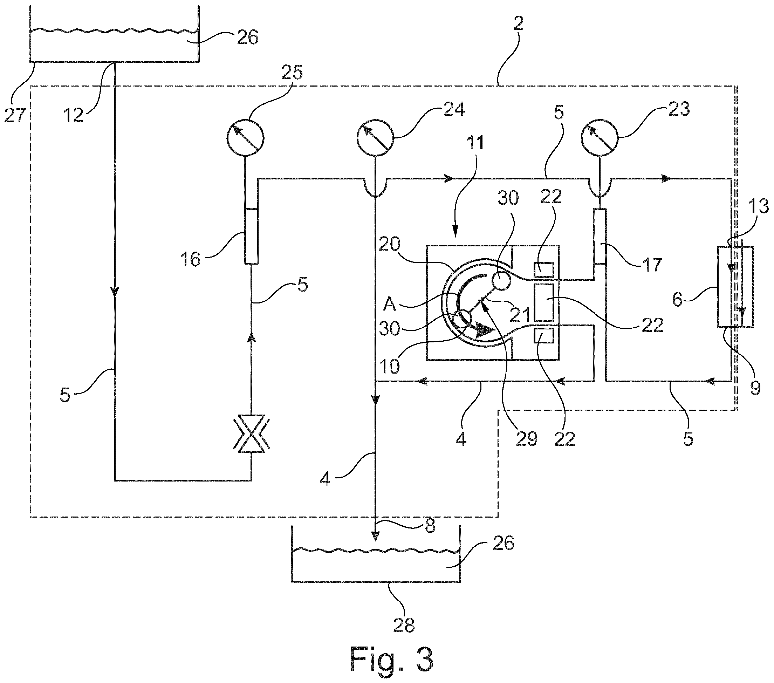

Figur 2 zeigt das in Figur 1 während einer Behandlung dargestellte Flüssigkeitssystem 1 während einer ersten Sequenz des Primens / der Vorbereitung zur Behandlung im Rahmen eines Verfahrens nach der Erfindung. Figur 3 zeigt das Flüssigkeitssystem 1 während einer zweiten, der ersten Sequenz nachfolgenden Sequenz des Primens / der Vorbereitung zur Behandlung im Rahmen eines Verfahrens nach der Erfindung. Anders als während der Behandlung sind während der Vorbereitung der arterielle patientenseitige Anschluss 8 und der venöse patientenseitige Anschluss 12 nicht mit der Blutbahn eines Patienten verbunden. Vielmehr ist der arterielle patientenseitige Anschluss 8 mit einem eine Primingflüssigkeit 26 enthaltenden Flüssigkeitsreservoir 27 und der venöse patientenseitige Anschluss 12 mit einem Brauchflüssigkeitsreservoir 28 verbunden. Das Flüssigkeitsreservoir 27 kann insbesondere ein interner Behälter 27 der Vorrichtung zur extrakorporalen Blutbehandlung mit einem Substituatport oder ein externer Behälter 27 mit Primingflüssigkeit sein. Das Brauchflüssigkeitsreservoir 28 kann insbesondere ein internes Brauchflüssigkeitsreservoir 28 mit einem Brauchflüssigkeitsanschluss (Wasteport) der Vorrichtung zur extrakorporalen Blutbehandlung oder ein separates Brauchflüssigkeitsreservoir 28, insbesondere ein Abfallbehälter oder Abfallbeutel, sein.

Eine in Figur 2 detailliert gezeigte Besonderheit des Verfahrens nach der Erfindung ist, dass bei der Vorbereitung / beim Primen das Pumpsegment 10 durch die Blutpumpe 11 nicht okkludiert ist und wird. Bei dem erfindungsgemäßen Verfahren wird zunächst das extrakorporale Blutsystem 2 komplett aufgerüstet. Das heißt, dass zunächst der filterseitige Anschluss 13 der venösen Blutleitung 5 sowie der filterseitige Anschluss 9 der arteriellen Blutleitung 4 an die Blutbehandlungseinheit 6 (Filter, Dialysator) angeschlossen werden. Danach werden der patientenseitige arterielle Anschluss 8 an das Brauchflüssigkeitsreservoir 28 und der patientenseitige venöse Anschluss 12 an das Flüssigkeitsreservoir 27 angeschlossen. Alternativ können die patientenseitigen Anschlüsse 8 bzw. 12 für ein automatisches Priming an den Substituatport bzw. den Wasteport der Maschine angeschlossen werden. Im vorliegenden Fall ist das Flüssigkeitsreservoir 27 ein einfacher Behälter 27 mit Kochsalzlösung als Primingflüssigkeit 26. Das Blutsystem 2 ist noch nicht mit Flüssigkeit gefüllt.

Das nicht mit Flüssigkeit gefüllte Pumpsegment 10 wird in die Haltekontur 22 der Blutpumpe 11 eingelegt und dabei nicht durch die Pumpe okkludiert. Alternativ kann das Pumpsegment 10 über den Rotor 21 der Blutpumpe 11 gehängt werden, derart, dass es am Rotor 21 gehalten, aber nicht zwischen Rotor 21 und Führungsfläche 20 eingequetscht und okkludiert wird. Nach einer weiteren Alternative kann es einfach über einen in den Figuren nicht gezeigten Haken oder Infusionsständer gehängt werden. Wesentlich ist, dass nicht okkludiert wird, also der Querschnitt der Blutleitung nicht verschlossen ist. Dadurch ist das Pumpsegment 10 während des Befüllvorgangs des Blutsystems 2 mit Primingflüssigkeit 26 durchgängig.

Nachfolgend wird das Blutsystem 2 vollständig mit Primingflüssigkeit 26 gefüllt. Das Befüllen erfolgt beispielsweise mittels Überdruck aus dem Substituatport (vorzugsweise mittels einer Substituatpumpe oder einer anderen Pumpe der Vorrichtung) oder mittels hydrostatischem Druck aus dem Kochsalzbehälter 27. Erst wenn das Blutsystem 2 komplett gefüllt ist, also die arterielle Blutleitung 4, die venöse Blutleitung 5, das Pumpsegment 10 und die Behandlungseinheit 6, kann das Pumpsegment 10 automatisch gegebenenfalls/wahlweise eingefädelt werden. Dies kann simpel durch Einschalten der Blutpumpe 10 erfolgen, wodurch der Rotor 21 die in der Haltekontur 22 gehaltene Leitung automatisch ergreifen kann und in der zum Fördern von Flüssigkeit bestimmten Weise zwischen sich und der Führungsfläche 20 platziert und okkludiert. Im weiteren Verlauf des Primens erfolgt die Förderung der Primingflüssigkeit 26 durch das Blutsystem 2 von venös nach arteriell beispielsweise durch eine Rückwärtsrotation der Blutpumpe 10, diese Drehrichtung ist in Figur 3 mittels des Pfeils A gekennzeichnet.

Bei der in den Figuren 2 und 3 gezeigten strömungstechnischen Verschaltung des Blutsystems 2 mit dem Flüssigkeitsreservoir 27 und dem Brauchflüssigkeitsreservoir 28 bzw. der Vorrichtung zur extrakorporalen Blutbehandlung wird in der ersten Sequenz der Vorbereitung, insbesondere beim Befüllen des Blutsystems mit Primingflüssigkeit, von venös nach arteriell geprimed, wie durch die in den Figuren 2 und 3 enthaltenen Strömungsrichtungspfeile angedeutet ist. Dies ist entgegen der üblicherweise meist beim Primen verwendeten Richtung. Durch Primen von venös nach arteriell kann Luft komplett aus der venösen Luftfalle 16 verdrängt werden, da diese von unten befüllt wird. Dies ist besonders vorteilhaft für sogenannte "luftfreie Blutleitungen". Es sei darauf hingewiesen, dass im Rahmen des erfindungsgemäßen Verfahrens in der zweiten Sequenz der Vorbereitung, insbesondere nach erfolgter Befüllung des Blutsystems mit Primingflüssigkeit, die Primingflüssigkeit in beide Strömungsrichtungen durch das System gefördert werden kann, auch wenn in Figur 3 Strömungspfeile nur in eine Richtung zeigen.

Ein nicht zwingend erforderlicher Verfahrensschritt besteht darin, vor dem eigentlichen Befüllen des Blutsystems mit Primingflüssigkeit einen Test der Sensoren 23, 24, 25 durchzuführen. Dazu können die arterielle Blutleitung 4 und die venöse Blutleitung 5 mittels in den Figuren nicht dargestellten arteriellen und venösen Absperrklemmen geschlossen werden. Mittels einer beispielsweise in die Maschine integrierten Pumpe kann dann der Luftdruck im Blutsystem durch Einströmen über einen Druckanschluss erhöht werden. Da alle Sensoren 23, 24, 25 miteinander hydraulisch über die nicht okkludierten Leitungen des extrakorporalen Blutsystems 2 verbunden sind, können sie gegeneinander getestet werden. Ein eventueller Offset aufgrund eines sich durch die Höhendifferenz ergebenden hydrostatischen Druckunterschieds ist dabei zu berücksichtigen.

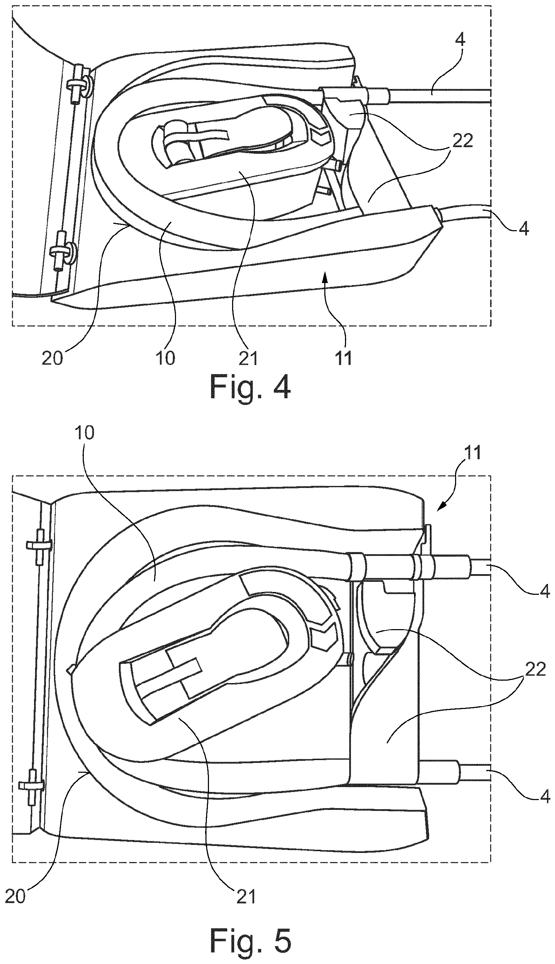

Figur 4 zeigt eine Detaildarstellung einer Blutpumpe 10 der Vorrichtung während der Vorbereitung. Pumpsegment 10 ist in die Haltekontur 22 eingelegt, aber noch nicht zwischen dem Rotor 21 und der Führungsfläche 20 okkludiert. Um im Blutsystem enthaltene Luft möglichst rückstandsfrei und vollständig verdrängen zu können, erfolgt das Befüllen der arteriellen Blutleitung 4 und des Pumpsegments 10 hier in vorteilhafter Weise von unten nach oben erfolgen, weshalb die Blutpumpe 11 derart an der Vorrichtung angeordnet und ausgerichtet ist, dass sie zur Seite hin geöffnet ist (siehe auch in den Figuren 1, 2 und 3).

Figur 5 zeigt eine Detaildarstellung der Blutpumpe 11 der Vorrichtung während der Blutbehandlung nach der ersten Sequenz des Priming, also nach dem vollständigen Füllen des Blutsystems mit Primingflüssigkeit. Der Pumpenrotor 21 wurde aus der in Figur 4 gezeigten Lage gedreht und das Pumpsegment 10 automatisch vom Rotor 21 mitgenommen und zwischen diesem und der Führungsfläche 20 eingefädelt. Mindestens eine Rolle des Rotors 21 ist danach mit dem Pumpsegment 10 in Eingriff und okkludiert dieses. Sobald das Pumpsegment 10 eingefädelt ist, kann die Blutpumpe 11 für die Förderung verwendet werden. Die im Blutsystem enthaltene Primingflüssigkeit kann darin zirkuliert werden, bis ein Patient angeschlossen und die Behandlung begonnen wird.

### Bezugszeichen

- 1: Flüssigkeitssystem
- 2: extrakorporales Blutsystem
- 3: Dialysierflüssigkeitskreislauf
- 4: arterielle Blutleitung
- 5: venöse Blutleitung
- 6: Blutbehandlungseinheit, Filter, Dialysator
- 7: Patient
- 8: patientenseitiger Anschluss, arterielle Kanüle (der arteriellen Blutleitung 4)
- 9: filterseitiger Anschluss, Luer-Konnektor (der arteriellen Blutleitung 4)
- 10: Pumpsegment
- 11: Blutpumpe, Schlauchpumpe
- 12: patientenseitiger Anschluss, venöse Kanüle (der venösen Blutleitung 5)
- 13: filterseitiger Anschluss, Luer-Konnektor (der venösen Blutleitung 5)
- 14: arterielle Luftfalle
- 15: Dialysierflüssigkeitspumpe
- 16: venöse Luftfalle
- 17: Bilanzierungseinrichtung
- 18: Ultrafiltrationspumpe
- 19: Pumpengehäuse
- 20: Führungsfläche
- 21: Rotor
- 22: Haltekontur
- 23: Eingangsdruckaufnehmer
- 24: arterieller Druckaufnehmer
- 25: venöser Druckaufnehmer
- 26: Primingflüssigkeit
- 27: Flüssigkeitsreservoir für Primingflüssigkeit
- 28: Brauchflüssigkeitsreservoir
- 29: Drehachse, Rotorachse
- 30: Anpresselement, Anpressrolle
- A: Rückwärtsdrehrichtung der Blutpumpe

## Patentansprüche

1. Verfahren zum Primen eines extrakorporalen Blutsystems (2), wobei das extrakorporale Blutsystem eine arterielle Blutleitung (4), eine venöse Blutleitung (5), ein Pumpsegment (10) zum Zusammenwirken mit einer peristaltischen Blutpumpe (11) einer Vorrichtung zur extrakorporalen Blutbehandlung und eine Blutbehandlungseinheit (6) aufweist,
wobei ein Anschluss (12, 13) der venösen Blutleitung (5) an einen venösen Blutanschluss der Blutbehandlungseinheit (6) angeschlossen wird, ein Anschluss (8, 9) der arteriellen Blutleitung (4) an einen arteriellen Blutanschluss der Blutbehandlungseinheit (6) angeschlossen wird und das extrakorporale Blutsystem (2) an ein Reservoir (27) mit Primingflüssigkeit (26) angeschlossen wird, und
nachfolgend das nicht okkludierte extrakorporale Blutsystem (2) mit Primingflüssigkeit (26) gefüllt wird
**dadurch gekennzeichnet, dass** das Pumpsegment (10) vor dem Füllen mit Primingflüssigkeit (26) derart in die Blutpumpe (11) eingelegt oder zu dieser positioniert wird, dass es während des Einfüllens von Primingflüssigkeit (26) in das Blutsystem (2) durch die Blutpumpe (11) nicht okkludiert wird und nach dem vollständigen Füllen des extrakorporalen Blutsystems (2) mit Primingflüssigkeit (26) automatisch durch Einschalten der Blutpumpe (11) in deren Förderstrecke platziert und okkludiert wird.

2. Verfahren nach Anspruch 1, **dadurch gekennzeichnet, dass** das extrakorporale Blutsystem (2) an das Reservoir (27) mit Primingflüssigkeit (26) angeschlossen wird, indem ein patientenseitiger Anschluss (12) der venösen Blutleitung (5) oder ein patientenseitiger Anschluss (8) der arteriellen Blutleitung (4) oder eine damit fluidisch verbundene Füllleitung an das Reservoir (27) mit Primingflüssigkeit (26) angeschlossen wird.

3. Verfahren nach Anspruch 1 oder 2, **dadurch gekennzeichnet, dass** der patientenseitige Anschluss (12) der venösen Blutleitung (5) an das Reservoir (27) mit Primingflüssigkeit (26) angeschlossen wird und nachfolgend das extrakorporale Blutsystem (2) von arteriell nach venös mit Primingflüssigkeit (26) gefüllt wird, wobei das Reservoir (27) ein Substituatport der Vorrichtung oder ein externer Behälter (27) mit Primingflüssigkeit (26) ist, oder dass der patientenseitige Anschluss (8) der arteriellen Blutleitung (4) an das Reservoir (27) mit Primingflüssigkeit (26) angeschlossen wird und nachfolgend das extrakorporale Blutsystem (2) von venös nach arteriell mit Primingflüssigkeit (26) gefüllt wird, wobei das Reservoir (27) ein Substituatport der Vorrichtung oder ein externer Behälter (27) mit Primingflüssigkeit (26) ist.

4. Verfahren nach einem der vorstehenden Ansprüche, **dadurch gekennzeichnet, dass** vor dem Füllen mit Primingflüssigkeit (26) ein patientenseitiger Anschluss (12) der venösen Blutleitung (5) oder ein patientenseitiger Anschluss (8) der arteriellen Blutleitung (4) mit einem Brauchflüssigkeitsanschluss der Vorrichtung zur extrakorporalen Blutbehandlung oder mit einem separaten Brauchflüssigkeitsreservoir (28) verbunden wird.

5. Verfahren nach einem der vorstehenden Ansprüche, **dadurch gekennzeichnet, dass** die Blutpumpe (11) während des Primens nach dem Befüllen mit Primingflüssigkeit in einer Förderrichtung betrieben wird, die der Förderrichtung während einer Blutbehandlung entgegengesetzt ist.

6. Verfahren nach einem der vorstehenden Ansprüche, **dadurch gekennzeichnet, dass** das Blutsystem (2) vor dem Füllen mit Primingflüssigkeit (26) in einer zur Behandlung bestimmten Weise an die Vorrichtung zur extrakorporalen Blutbehandlung angeschlossen wird, mit der Ausnahme, dass das Pumpsegment (10) nicht okkludiert wird.

7. Verfahren nach einem der vorstehenden Ansprüche, **dadurch gekennzeichnet, dass** vor dem Füllen des extrakorporalen Blutsystems (2) mit Primingflüssigkeit (26) ein Sensortestlauf durchgeführt wird, indem die arterielle Blutleitung (4) und/oder die venöse Blutleitung (5) verschlossen werden und der Druck im extrakorporalen Blutsystem (2) erhöht wird.

8. Verfahren nach einem der vorstehenden Ansprüche, **dadurch gekennzeichnet, dass** die Primingflüssigkeit (26) mittels Überdruck auf einem Substituatport der Vorrichtung oder hydrostatischem Druck in das extrakorporale Blutsystem (2) gefüllt wird.

9. Vorrichtung zur extrakorporalen Blutbehandlung mit einer peristaltischen Blutpumpe (11) zum Fördern von Flüssigkeit durch peristaltisches Verformen eines Pumpsegments (10) eines extrakorporalen Blutsystems (2) mit einer arteriellen Blutleitung (4), einer venösen Blutleitung (5), dem Pumpsegment (10) und einer Blutbehandlungseinheit (6), welche Vorrichtung eingerichtet ist, das Verfahren nach einem der vorhergehenden Ansprüche auszuführen, wobei die Blutpumpe eine teilkreisförmige Führungsfläche und einen um eine im Wesentlichen horizontale Drehachse drehangetriebenen Rotor aufweist, der derart mit der Führungsfläche zusammenwirkt, dass in dem zwischen der Führungsfläche und dem Rotor angeordneten Pumpsegment eine Querschnittsverengung ausbildet wird, **dadurch gekennzeichnet, dass** sich die Führungsfläche (20) von einem Bereich unterhalb des Rotors (21) teilweise um diesen herum bis zu einem Bereich oberhalb des Rotors (21) erstreckt und zur Seite hin offen ist und die Vorrichtung und/oder die Blutpumpe (11) zumindest eine Haltevorrichtung (22) aufweisen, zum nicht-okkludierenden Halten des Pumpsegments (10) in einer ein automatisches Einfädeln des Pumpsegments (10) in die Blutpumpe (11) ermöglichenden Weise.

10. Vorrichtung nach Anspruch 9, **dadurch gekennzeichnet, dass** der Rotor (21) zum Okkludieren des extrakorporalen Blutsystems (2) derart angetrieben und/oder angesteuert ist, dass er relativ zur Führungsfläche (20) in einer Richtung quer zu seiner Drehachse positioniert und in dieser Position gehalten ist und/oder zur Rotordrehachse in radialer Richtung relativpositionierbare Anpresselemente aufweist.

## Claims

1. A method of priming an extracorporeal blood system (2), the extracorporeal blood system comprising an arterial blood line (4), a venous blood line (5), a pump segment (10) for interaction with a peristaltic blood pump (11) of an apparatus for extracorporeal blood treatment and comprising a blood treatment unit (6), wherein a port (12, 13) of the venous blood line (5) is connected to a venous blood port of the blood treatment unit (6), a port (8, 9) of the arterial blood line (4) is connected to an arterial blood port of the blood treatment unit (6) and the extracorporeal blood system (2) is connected to a reservoir (27) containing priming liquid (26), and
subsequently the non-occluded extracorporeal blood system (2) is filled with priming liquid (26),
**characterized in that** prior to filling with priming liquid (26), the pump segment (10) is inserted into or positioned relative to the blood pump (11) so that during filling of priming liquid (26) into the blood system (2) it is not occluded by the blood pump (11) and, after complete filling of the extracorporeal blood system (2) with priming liquid (26), is automatically placed and occluded in the delivery path of the blood pump (11) by activating the latter.

2. The method according to claim 1, **characterized in that** the extracorporeal blood system (2) is connected to the reservoir (27) containing priming liquid (26), as a patient-side port (12) of the venous blood line (5) or a patient-side port (8) of the arterial blood line (4) or a filling line being fluid-connected thereto is connected to the reservoir (27) containing priming liquid (26).

3. The method according to claim 1 or 2, **characterized in that** the patient-side port (12) of the venous blood line (5) is connected to the reservoir (27) containing priming liquid (26) and subsequently the extracorporeal blood system (2) is filled from the arterial side to the venous side with priming liquid (26), wherein the reservoir (27) is a substituate port of the apparatus or an external container (27) containing priming liquid (26), or **in that** the patient-side port (8) of the arterial blood line (4) is connected to the reservoir (27) containing priming liquid (26) and subsequently the extracorporeal blood system (2) is filled from the venous side to the arterial side with priming liquid (26), wherein the reservoir (27) is a substituate port of the apparatus or an external container (27) containing priming liquid (26).

4. The method according to any one of the preceding claims, **characterized in that,** prior to filling with priming liquid (26), a patient-side port (12) of the venous blood line (5) or a patient-side port (8) of the arterial blood line (4) is connected to a process liquid port of the apparatus for extracorporeal blood treatment or to a separate process liquid reservoir (28).

5. The method according to any one of the preceding claims, **characterized in that** during priming after filling with priming liquid the blood pump (11) is operated in a delivery direction which is opposed to the delivery direction during blood treatment.

6. The method according to any one of the preceding claims, **characterized in that,** prior to filling with priming liquid (26), the blood system (2) is connected to the apparatus for extracorporeal blood treatment in a manner intended for treatment, with the exception that the pump segment (10) is not occluded.

7. The method according to any one of the preceding claims, **characterized in that,** prior to filling of the extracorporeal blood system (2) with priming liquid (26), a sensor test run is carried out, by closing the arterial blood line (4) and/or the venous blood line (5) and the pressure is increased in the extracorporeal blood system (2).

8. The method according to any one of the preceding claims, **characterized in that** the priming liquid (26) is filled into the extracorporeal blood system (2) by means of excess pressure on a substituate port of the apparatus or hydrostatic pressure.

9. An apparatus for extracorporeal blood treatment comprising a peristaltic blood pump (11) for delivering liquid by peristaltic deformation of a pump segment (10) of an extracorporeal blood system (2) comprising an arterial blood line (4), a venous blood line (5), the pump segment (10) and a blood treatment unit (6), wherein the apparatus is configured to perform the method according to one of the preceding claims, wherein the blood pump has a partially circular guiding surface and a rotor being rotationally driven about a substantially horizontal axis of rotation and interacting with the guiding surface such that in the pump segment disposed between the guiding surface and the rotor a cross-sectional constriction is formed, **characterized in that** the guiding surface (20) extends from an area beneath the rotor (21) partially around the latter up to an area above the rotor (21) and is open towards the side and the apparatus and/or the blood pump (11) include(s) at least one retaining device (22) for retaining the pump segment (10) in a non-occluding manner in a way which enables the pump segment (10) to be automatically threaded into the blood pump (11).

10. The apparatus according to claim 9, **characterized in that** the rotor (21) is driven and/or controlled for occluding the extracorporeal blood system (2) in such a way that it is positioned relative to the guiding surface (20) in a direction transversely to its axis of rotation and is held in said position and/or includes pressing elements which can be positioned relative to the axis of rotation of the rotor in the radial direction.

## Revendications

1. Procédé pour amorcer un système sanguin extracorporel (2), dans lequel le système sanguin extracorporel présente un vaisseau sanguin artériel (4), un vaisseau sanguin veineux (5), un segment de pompe (10) pour la coopération avec une pompe à sang (11) péristaltique d'un dispositif de traitement extracorporel du sang et une unité de traitement du sang (6),
dans lequel un raccord (12, 13) du vaisseau sanguin veineux (5) est raccordé à un raccord sanguin veineux de l'unité de traitement du sang (6), un raccord (8, 9) du vaisseau sanguin artériel (4) est raccordé à un raccord sanguin artériel de l'unité de traitement du sang (6) et le système sanguin extracorporel (2) est raccordé à un réservoir (27) avec du liquide d'amorce (26), et
le système sanguin extracorporel (2) non obstrué est par la suite rempli de liquide d'amorce (26),
**caractérisé en ce que** le segment de pompe (10) est inséré avant le remplissage avec du liquide d'amorce (26) dans la pompe à sang (11) ou est positionné par rapport à celle-ci de telle manière qu'il ne soit pas obstrué pendant le remplissage du liquide d'amorce (26) dans le système sanguin (2) par la pompe à sang (11) et est placé et obstrué après le remplissage complet du système sanguin extracorporel (2) avec du liquide d'amorce (26) automatiquement par la mise en service de la pompe à sang (11) dans sa ligne de refoulement.

2. Procédé selon la revendication 1, **caractérisé en ce que** le système sanguin extracorporel (2) est raccordé au réservoir (27) avec du liquide d'amorce (26), **en ce qu'**un raccord (12) côté patient du vaisseau sanguin veineux (5) ou un raccord (8) côté patient du vaisseau sanguin artériel (4) ou une conduite de remplissage reliée fluidiquement à celui-ci est raccordée au réservoir (27) avec du liquide d'amorce (26).

3. Procédé selon la revendication 1 ou 2, **caractérisé en ce que** le raccord coté patient (12) du vaisseau sanguin veineux (5) est raccordé au réservoir (27) avec du liquide d'amorce (26) et le système sanguin extracorporel (2) est par la suite rempli du vaisseau sanguin artériel au vaisseau sanguin veineux avec du liquide d'amorce (26), dans lequel le réservoir (27) est un orifice substitut du dispositif ou un récipient externe (27) avec du liquide d'amorce (26), ou que le raccord (8) côté patient du vaisseau sanguin artériel (4) est raccordé au réservoir (27) avec du liquide d'amorce (26) et le système sanguin extracorporel (2) est par la suite rempli du vaisseau sanguin veineux au vaisseau sanguin artériel avec du liquide d'amorce (26), dans lequel le réservoir (27) est un orifice substitut du dispositif ou un récipient externe (27) avec du liquide d'amorce (26).

4. Procédé selon l'une quelconque des revendications précédentes, **caractérisé en ce que** avant le remplissage avec du liquide d'amorce (26), un raccord (12) côté patient du vaisseau sanguin veineux (5) ou un raccord (8) côté patient du vaisseau sanguin artériel (4) est relié à un raccord de liquide non potable du dispositif de traitement extracorporel du sang ou à un réservoir de liquide non potable (28) séparé.

5. Procédé selon l'une quelconque des revendications précédentes, **caractérisé en ce que** la pompe à sang (11) est actionnée pendant l'amorce après le remplissage avec du liquide d'amorce dans un sens de refoulement qui est opposé au sens de refoulement pendant un traitement du sang.

6. Procédé selon l'une quelconque des revendications précédentes, **caractérisé en ce que** le système sanguin (2) est raccordé avant le remplissage avec du liquide d'amorce (26) d'une manière destinée au traitement au dispositif de traitement extracorporel du sang, sauf si le segment de pompe (10) n'est pas obstrué.

7. Procédé selon l'une quelconque des revendications précédentes, **caractérisé en ce que** avant le remplissage du système extracorporel sanguin (2) avec du liquide d'amorce (26), un essai de capteur est mené **en ce que** le vaisseau sanguin artériel (4) et/ou le vaisseau sanguin veineux (5) est fermé et la pression est augmentée dans le système sanguin extracorporel (2).

8. Procédé selon l'une quelconque des revendications précédentes, **caractérisé en ce que** le liquide d'amorce (26) est rempli au moyen de la surpression sur un orifice substitut du dispositif ou de la pression hydrostatique dans le système sanguin extracorporel (2).

9. Dispositif de traitement extracorporel du sang avec une pompe à sang (11) péristaltique pour le refoulement de liquide par déformation péristaltique d'un segment de pompe (10) d'un système sanguin extracorporel (2) avec un vaisseau sanguin artériel (4), un vaisseau sanguin veineux (5), le segment de pompe (10) et une unité de traitement du sang (6), lequel dispositif est conçu afin de réaliser le procédé selon l'une quelconque des revendications précédentes, dans lequel la pompe à sang présente une surface de guidage partiellement circulaire et un rotor entraîné en rotation autour d'un axe de rotation sensiblement horizontal qui coopère avec la surface de guidage de telle manière que dans le segment de pompe agencé entre la surface de guidage et le rotor, un rétrécissement de section soit formé, **caractérisé en ce que** la surface de guidage (20) s'étend d'une zone en dessous du rotor (21) partiellement autour de celui-ci jusqu'à une zone au-dessus du rotor (21) et est ouverte vers le côté et le dispositif et/ou la pompe à sang (11) présente au moins un dispositif de retenue (22) pour la retenue non obstruante du segment de pompe (10) d'une manière permettant un embobinage automatique du segment de pompe (10) dans la pompe à sang (11).

10. Dispositif selon la revendication 9, , **caractérisé en ce que** le rotor (21) est entraîné et/ou commandé pour l'occlusion du système sanguin extracorporel (2) de telle manière qu'il soit positionné par rapport à la surface de guidage (20) dans un sens transversalement à son axe de rotation et soit maintenu dans cette position et/ou présente des éléments de pressage positionnables relativement par rapport à l'axe de rotation de rotor dans le sens radial.
